(19) **Europäisches Patentamt**

**European Patent Office**

**Office européen des brevets**

(11) **EP 0 693 135 B1**

(12) **FASCICULE DE BREVET EUROPEEN**

(45) Date de publication et mention
de la délivrance du brevet:
**08.03.2000 Bulletin 2000/10**

(51) Int. Cl.⁷: **C12Q 1/68**, G01N 27/00

(21) Numéro de dépôt: **94913139.5**

(22) Date de dépôt: **06.04.1994**

(86) Numéro de dépôt international:
**PCT/FR94/00382**

(87) Numéro de publication internationale:
**WO 94/23065 (13.10.1994 Gazette 1994/23)**

(54) **PROCEDE RAPIDE DE DETERMINATION D'UNE SEQUENCE D'ADN ET APPLICATION AU SEQUEN AGE ET AU DIAGNOSTIC**

**SCHNELLES VERFAHREN FUR DIE DNS SEQUENZIERUNG UND DIAGNOSTISCHE ANWENDUNGEN**

**FAST DNA SEQUENCE DETERMINATION METHOD AND USE THEREOF IN SEQUENCING AND DIAGNOSTICS**

(84) Etats contractants désignés:
**AT BE CH DE DK ES FR GB GR IE IT LI LU MC NL PT SE**

(30) Priorité: **06.04.1993 FR 9304056**

(43) Date de publication de la demande:
**24.01.1996 Bulletin 1996/04**

(73) Titulaires:
- **INSTITUT PASTEUR**
  **75015 Paris (FR)**
- **CENTRE NATIONAL DE LA RECHERCHE SCIENTIFIQUE**
  **75794 Paris Cédex 16 (FR)**

(72) Inventeurs:
- **BENSIMON, Aaron**
  **F-75015 Paris (FR)**
- **BENSIMON, David**
  **F-75013 Paris (FR)**
- **CROQUETTE, Vincent**
  **F-92160 Antony (FR)**
- **CHIFFAUDEL, Arnaud**
  **F-92160 Antony (FR)**

(74) Mandataire: **Warcoin, Jacques**
  **Cabinet Régimbeau,**
  **26, avenue Kléber**
  **75116 Paris (FR)**

(56) Documents cités:
**EP-A- 0 397 416**

- **SCIENCE vol. 258 , Novembre 1992 , LANCASTER, PA US pages 1122 - 1126 S.SMITH ET AL. 'Direct mechanical measurements of the elasticity of single DNA molecules by using magnetic beads' cité dans la demande**

**Description**

La présente invention concerne un procédé de détermination rapide d'une séquence d'un acide nucléique, ADN ou ARN, utile notamment pour le séquençage d'un ADN ou ARN inconnu ou bien pour la détection d'une séquence d'ADN ou ARN spécifique pour le diagnostic.

Le séquençage de l'ADN est un objectif majeur de la biologie moléculaire et se trouve au centre du projet de séquençage du génome humain.

De plus, la mise en évidence de la présence d'une séquence d'ADN spécifique dans un échantillon physiologique constitue, actuellement, l'axe de développement majeur des méthodes de diagnostic. Après la technique immunologique, on se tourne maintenant vers des méthodes de diagnostic menant en évidence les modifications de l'ADN lui-même, ceci afin de prévenir notamment la- résistance aux antibiotiques (voir Nature, 1992, 358, p. 591) ,les anomalies génétiques, les risques de cancer liés à des modifications génétiques et les infections virales, par exemple les infections liées à HIV ou aux virus des hépatites.

Ces méthodes de diagnostic comprennent actuellement les méthodes dites "d'hybridation directe" qui vont détecter la présence de cette séquence par hybridation directe d'une sonde avec l'échantillon. La méthode est lourde et peu précise, notamment car elle nécessite une détection par gel et exposition sur film photosensible ou comptage radioactif.

Les méthodes utilisant une amplification, notamment la méthode PCR, qui avant la mise en évidence de la séquence par hybridation, vont d'abord amplifier la partie correspondante de l'ADN en utilisant par exemples des amorces et une polymérase, sont excessivement sensibles aux contaminations.

Ces méthodes présentent, en outre, l'inconvénient d'être des systèmes de reconnaissance indirects parce que la séquence recherchée n'est reconnue que par l'intermédiaire d'une sonde.

Le procédé selon la présente invention permet la mise en évidence de l'hybridation ou de la séquence nucléotidique si cela est nécessaire. Il ne peut donc y avoir de biais ou d'ambiguité. Ce progrès considérable est dû au fait que, pour la première fois, il est presqu'aussi rapide de mettre en évidence directement la structure nucléotidique de la séquence recherchée que de la meure en évidence indirectement, à l'aide d'une sonde par exemple.

Il est même possible, grâce au procédé selon l'invention, de mettre en évidence directement l'hybridation entre deux séquences complémentaires sans avoir recours au séquençage complet de cette séquence.

Le séquençage des acides nucléiques est aujourd'hui essentiellement réalisés par la méthode dite Sanger bien connue des biologistes. Dans cette technique l'ADN à séquencer est d'abord découpé en petits segments (d'environ 5 kb), qui sont ensuite clonés dans des plasmides insérés dans une souche bactérienne. Les plasmides sont amplifiés dans cette souche et extraits. L'ADN inséré est alors copié à partir d'un promoteur plasmidique. Cette transcription est interrompue après l'insertion d'un nombre aléatoire de nucléotides. On possède ainsi une population de toutes les copies interrompues possibles de l'ADN initial. Cette population est séparée sur gel par électrophorèse selon le poids moléculaire des multiples copies. La position des nucléotides le long de l'ADN est lue directement sur le gel pour chaque fragment d'ADN.

Ce processus est très laborieux. Pour un ADN d'environ 15 000 paires de bases (15 kbp) la dernière étape (après l'extraction des plasmides) prend à elle seule une semaine. En conséquence, on admet couramment que le séquençage d'un ADN revient à environ 5 francs par paire de base à la date de dépôt de la présente demande.

Le procédé selon la présente invention, reposant sur des techniques physiques et des traitements électroniques, se distingue des approches actuelles qui sont chimiques ou biochimiques. Ses avantages sont multiples :

1) Il permet aussi bien le séquençage de séquences courtes que le séquençage de gènes très longs (supérieurs à 10 kbp), sans avoir à les segmenter en petits morceaux, comme c'est le cas dans les méthodes actuelles.

2) Il est 10 000 fois plus rapide. Les méthodes biochimiques actuelles permettent de séquencer environ 15 000 bases en une semaine. Le procédé permet d'en séquencer environ 100 en une seconde, c'est-à-dire 60 millions en une semaine.

3) Il requiert une très faible quantité d'ADN.

4) Il peut être adapté facilement et présente de nombreux degrés de liberté.

5) Il est aisément automatisable, le signal de séquençage pouvant être directement traité sur ordinateur.

6) Il est potentiellement bien moins onéreux que la technique actuelle, car il n'exige pas de longues et laborieuses manipulations biochimiques. Comme le temps de séquençage est une partie déterminante de son coût, le procédé devrait être au moins 1 000 fois moins cher.

La présente invention concerne un procédé de détermination d'une séquence d'ADN ou d'ARN, caractérisé en ce qu'on détermine l'énergie d'appariement ou de desappariement entre chaque paire de base de l'ADN ou d'un hybride ARN/ADN double brin correspondant à la séquence d'ADN ou d'ARN à déterminer et en ce que l'on compare cette énergie à des valeurs prédéterminées.

Ce procédé présente plusieurs modes de mise en oeuvre. Dans le premier mode, on peut affecter à chaque énergie mesurée une valeur prédéterminée qui correspond à une paire de base en présence de bases avoisinantes correspondantes, on reconstitue ainsi la séquence nucléotidique de l'ADN ou de l'ARN en cause.

Dans un second mode de mise en oeuvre du procédé, on cherche à mettre en évidence la présence d'une séquence connue. Dans ce cas on peut avoir simplement déterminé les éléments essentiels de cette séquence recherchée pour en faire une "empreinte" et on peut alors se contenter de contrôler que l'échantillon d'ADN ou d'ARN à déterminer présente la même empreinte ou non.

Dans un troisième mode de mise en oeuvre du procédé, on détermine l'énergie globale nécessaire pour désapparier l'ADN double brin ou l'hybride ADN/ARN double brin et on compare cette énergie globale à une valeur de seuil de cette énergie correspondant aux mêmes séquences totalement appariées, ce qui permet d'écarter des complexes ADN/ADN ou ARN/ADN non totalement appariés dont l'énergie de désappariement est inférieure à la valeur de seuil ; on appellera "l'empreinte" en cause "empreinte globale", ou signal.

Ces deux derniers modes de mise en oeuvre du procédé sont plus particulièrement destinés au diagnostic, c'est-à-dire à la mise en évidence de la présence ou de l'absence d'une séquence connue, alors que le premier mode de mise en oeuvre est plus particulièrement destiné au séquençage.

Par "énergie d'appariement ou de désappariement" on entend désigner essentiellement l'énergie nécessaire pour séparer ou récupérer en reformant ("réapparier") deux paires de bases appariées sur deux brins d'ADN complémentaires ou d'un hybride ADN/ARN.

L'invention repose sur la mise en évidence qu'il est possible de mesurer, de façon rapide et fiable, la force-de liaison de chaque paire de base d'un ADN double brin ou d'un hybride ADN/ARN et d'affecter à chacune de ces valeurs une séquence spécifique, la force de liaison dépendant non seulement de la paire de base en cause mais également de son environnement, notamment des bases avoisinantes et éventuellement des conditions expérimentales dans lesquelles se déroulent l'appariement ou le désappariement.

En effet, l'ADN est une double hélice formée par deux brins composés des acides nucléiques adénine, thymine, guanine et cytosine, dont la cohésion est réalisée, d'une part, par les liaisons hydrogènes entre les paires de bases adénine (A)/thymine (T) -deux liaisons- et les paires guanine (G)/cytosine (C) -trois liaisons- ; d'autre part, par les liaisons dues aux énergies d'empilement des différentes paires de bases. Le tableau 1 donne une idée des énergies correspondantes pour l'ADN. On constate que ces énergies varient notablement en fonction des paires de base et des bases avoisinantes. Les résultats de ce tableau correspondent aux énergies mesurées pour désapparier des dimères formés de deux bases contiguës. On peut se référer également à l'ouvrage de W. Saenger (Principles of Nucleic Acid structure 1988, édité par Springer-Verlag). Les flèches du tableau représentent le sens de lecture de l'ADN.

TABLEAU 1

| Dimères | Energies (kJ x mole$^{-1}$ x dimère$^{-1}$) |
|---|---|
| ↑ C.G │ <br> │ G.C │ ↓ | - 60,99 |
| ↑ C.G │ ↑ T.A │ <br> │ A.T ↓ │ G.C ↓ | - 43,93 |
| ↑ C.G │ ↑ A.T │ <br> │ T.A ↓ │ G.C ↓ | - 41,01 |
| ↑ G.C │ <br> │ C.G ↓ | - 40,50 |
| ↑ G.C │ ↑ C.G │ <br> │ G.C ↓ │ C.G ↓ | - 34,53 |
| ↑ T.A │ <br> │ A.T ↓ | - 27,46 |
| ↑ G.C │ ↑ A.T │ <br> │ T.A ↓ │ C.G ↓ | - 27,46 |
| ↑ G.C │ ↑ T.A │ <br> │ T.A ↓ │ C.G ↓ | - 28,34 |
| ↑ A.T │ ↑ T.A │ <br> │ A.T ↓ │ T.A ↓ | - 22,45 |
| ↑ A.T │ <br> │ T.A ↓ | - 15,97 |

Ce procédé comporte deux variantes essentielles.

Dans le premier mode de mise en oeuvre on détermine l'énergie de desappariement entre chaque paire de base de l'ADN ou d'hybride ADN/ARN par fixation d'au moins une base de chaque brin de l'ADN ou de l'hybride ADN/ARN sur un support et éloignement des supports afin d'arracher, l'une après l'autre, chaque paire de base en mesurant à chaque desappariement l'énergie nécessaire au desappariement et en la comparant avec des valeurs prédéterminées.

Dans le second mode de mise en oeuvre on détermine l'énergie d'appariement entre chaque paire de base de l'ADN ou d'hybride ADN/ARN par fixation d'au moins une base de chaque brin de l'ADN ou de l'hybride ADN/ARN sur un support et rapprochement des supports afin d'apparier chaque paire de base en mesurant à chaque appariement l'énergie nécessaire à l'appariement et en la comparant avec des valeurs prédéterminées.

La fixation de chaque brin d'ADN à un support par au moins une base peut être réalisée par fixation directe de ladite base sur le support mais également par fixation indirecte par l'intermédiaire d'un bras qui peut être par exemple un peptide ou une chaîne carbonée inerte, ceci pour éviter les effets liés à la surface du support.

Il est également possible de fixer à un support un segment d'ADN ou d'ARN par plusieurs bases.

Enfin, on préférera fixer chaque brin de l'ADN par des bases contiguës, mais dans certains cas, en particulier lorsque l'on ne recherche qu'un signal correspondant à une "empreinte globale", on peut fixer chaque brin d'ADN par des bases situées à des extrémités opposées.

Bien entendu, il doit être compris que lorsque l'on utilise les termes "énergie d'appariement ou de desappariement" il s'agit de déterminer cette énergie, soit directement, soit indirectement par le biais d'une grandeur. Il peut s'agir notamment d'une force (notamment gravitationnelle ou magnétique) ou d'un temps ou d'une température d'appariement ou de desappariement. Certaines de ces grandeurs peuvent être liées au déplacement d'un ressort, d'une membrane ou, dans certains cas, à des variations de paramètres électriques ou magnétiques.

Si l'on souhaite se représenter schématiquement l'ADN double brin dans le cadre de la présente invention, il est possible de l'assimiler à une "fermeture éclair" que l'on ouvre (ou que l'on ferme) en mesurant pour chaque élément l'énergie mise en oeuvre lors du désappariement (ou appariement).

Le procédé selon l'invention peut être utilisé pour différentes applications.

Il peut, tout d'abord, être utilisé pour le séquençage direct d'un acide nucléique inconnu. Dans ce cas les valeurs prédéterminées des énergies d'appariement et de désappariement sont mesurées au préalable pour chaque paire de base en fonction de leur environnement et on peut alors directement affecter l'une des bases possibles à chaque désappariement ou appariement en fonction de l'énergie mesurée.

Dans ce type de séquençage, il peut être intéressant, après arrachement des deux brins, de les réapparier à nouveau puis de mesurer de nouveau les valeurs des énergies de liaison afin d'accumuler les données et diminuer ainsi le bruit de fond.

On peut également, pour faciliter le réappariement, prévoir que les extrémités libres de l'ADN double brin avant l'arrachement sont reliées entre elles de manière covalente ou quasi covalente, ce qui permet notamment d'effectuer des cycles d'appariement et de desappariement et d'améliorer ainsi le rapport signal/bruit.

Les techniques permettant de solidariser les extrémités libres de l'ADN double brin sont connues et certaines seront décrites plus en détail dans ce qui va suivre.

Le procédé selon la présente invention peut également être utilisé aux fins de diagnostic pour permettre notamment le séquençage de régions variables de l'ADN correspondant à des anomalies recherchées, la technique est alors similaire à celle décrite précédemment pour le séquençage.

Mais il est possible de prévoir une technique simplifiée dans laquelle les valeurs des forces sont enregistrées sous forme d'une "empreinte" ou signal qui sera comparé avec l'empreinte ou le signal recherché sans que l'ordre et la nature des bases soient expressément déterminés. Si l'empreinte recherchée correspond à la présence d'une anomalie, toute autre empreinte sera considérée comme négative ou bien au contraire on pourra retenir une empreinte correspondant à l'ADN normal en considérant comme positive toute autre empreinte.

A des fins de diagnostic, une variante plus simple du procédé décrit précédemment peut être utilisée. Il s'agit non pas de mesurer séquentiellement l'énergie nécessaire au desappariement des paires de bases, mais l'énergie totale nécessaire au desappariement de deux segments hybridés en tirant sur leurs extrémités. Dans ce but, une sonde complémentaire à la séquence cible cherchée dans un échantillon d'ADN/ARN est greffée sur un support (par des méthodes connues). L'ADN/ARN simple brin de l'échantillon est alors hybridé avec la sonde, puis l'une de ses extrémités ancrée sur une deuxième surface adéquate (par exemple une bille magnétique recouverte de streptavidine permettant une liaison quasi covalente avec un ADN préalablement biotinylé). L'énergie totale nécessaire pour "déchirer" les deux segments hybridés sert d' "empreinte" permettant l'identification de l'hybridation.

La mise en oeuvre du procédé a été rendue possible notamment par l'existence du microscope à force atomique (AFM) qui permet de mesurer directement les forces, comme cela ressortira de la description.

Le microscope à force atomique (AFM) est un appareil commercialisé par différentes compagnies (Park, Digital, etc.). Il sert en général à visualiser une surface en la balayant avec une pointe très fine placée au bout d'un levier.

Ainsi, l'un des moyens les plus simples pour tirer sur l'ADN et pour mesurer la force exercée consiste à utiliser un microscope à force atomique (AFM), en attachant l'une des extrémités de la molécule d'ADN à la pointe ou au levier de l'AFM et en liant l'autre à la surface porte échantillon solidaire du tube piezoélectrique mobile de l'AFM. Ces procédés permettant la fixation de l'ADN sur des surfaces sont connus et certaines des méthodes utilisables sont rappelées ci-après.

Bustamante et al. ont décrit dans Science (1992, vol. 258, p. 1122) une méthode de mesure de l'élasticité de l'ADN double brin mettant en oeuvre un moyen d'étirement différent de l'ADN, basé sur des forces visqueuses et magnétique.

Le principe même de cette méthode ne peut s'appliquer au séquençage car il n'y a pas séparation des deux brins d'ADN et la résolution spatiale est insuffisante (de l'ordre du micron).

En modifiant la tension électrique appliquée sur le tube de l'AFM, le tube se déplace et peut ainsi entraîner l'une des extrémités de l'ADN si celui-ci a été préalablement fixé d'un côté au levier et de l'autre au support. La traction ainsi imposée sur les bases appariées est transmise au levier de l'AFM, dont la déflection permet une mesure précise de cette force. La constante élastique typique du levier d'un AFM étant d'environ $3.10^{-2}$ N/m et la précision sur la mesure de déflection du levier pouvant atteindre par exemple 0.2 Å, la précision sur la force mesurée est meilleure que $0,6.10^{-12}$ N, ce qui est environ 100 fois plus faible que les forces d'appariement inter-brins (réalisés par des liaisons hydrogène). On peut se référer à l'article de J. N. Israelachvili (Intermolecular and Surface Forces, édité par Academic Press, 1985). L'AFM est un outil commode pour mesurer ce type de force, mais il n'est pas indispensable et peut être remplacé par tout autre méthode sensible de mesure de force, telle que les méthodes de détection capacitive ou par lévitation magnétique. Les mesures de forces par cette dernière méthode sont décrites par B. Gauthier-Manuel, Europhys. Conf. 14C C34, 199O.

La description ci-après est faite en se référant à l'AFM mais est adaptable à tout autre dispositif.

La périodicité des paires de bases le long de la molécule d'ADN est de 3,4 Å et permet de limiter l'influence des parasites. En tirant sur les deux extrémités contiguës des brins d'ADN, la force mesurée aura une composante périodique (de période environ 6,8 Å), modulée par les forces de cohésion interbrins qui reflètent la séquence des acides nucléiques dans l'ADN. Cette composante périodique peut donc être utilisée pour le séquençage de l'ADN après calibration de ces modulations avec des séquences connues d'ADN ou d'ARN mais également permet d'éliminer les parasites.

En effet, la force de traction sur le levier dépend de divers effets parasites (par exemple les forces de Van-der Waals entre le levier et la surface, etc.), en plus de la force de traction due au déroulement de l'ADN. Cependant, seule la force de traction de la molécule d'ADN sur le levier est périodique avec une période d'environ 6,8 Å. Les techniques de filtrage du signal permettent d'éliminer les effets parasites ayant des fréquences différentes. En calibrant, une fois pour toutes, le signal de force mesuré pour des séquences connues d'ADN, on établit une correspondance entre l'amplitude de la force périodique et la séquence de l'ADN. La mesure de l'amplitude du signal périodique peut alors être utilisée pour séquencer des fragments d'ADN inconnus. A la fin du cycle de traction, la molécule restant ancrée aux surfaces, on peut aussi mesurer un signal de force périodique provenant du réappariement des paires de bases lorsqu'on rapproche les deux brins (en "refermant la fermeture éclair"). Ainsi, en répétant les cycles d'étirement/rapprochement (traction/relaxation) on peut améliorer le rapport signal/bruit et la fiabilité du séquençage.

Enfin, comme cela a été précisé précédemment, il est possible, afin de faire varier les énerges mises en jeu, de faire varier les conditions de mise en oeuvre du procédé, en particulier les conditions du milieu où ont lieu appariement et désappariement. Ainsi, en modifiant le pH, la température, la force ionique ou la nature des produits ajoutés, on peut modifier les valeurs des énergies mises en jeu pour une même paire de base. Dans ce cas, évidemment, il pourra être nécessaire d'effectuer les mesures de référence dans les mêmes conditions pour l'étalonnage. Toutefois, les valeurs d'étalonnage pourront dans certains cas être obtenues par le calcul ou à l'aide d'abaques ou de tables.

Afin de fixer l'ADN sur les surfaces ou supports, on peut avoir recours à l'une quelconque des techniques connues dans le domaine. On peut distinguer essentiellement deux techniques :

1°) La méthode directe

Dans cette méthode l'ADN vient s'ancrer directement sur le support, par exemple la pointe de l'AFM, ce qui implique une fonctionnalisation de cette surface. Par exemple en la recouvrant de streptavidine, de groupement COOH, etc. capables de réagir avec l'extrémité fonctionnalisée de l'ADN.

2°) La méthode indirecte

L'ADN est ancré à une particule, par exemple poudre ou microbille adéquate (éventuellement magnétique), elle-même liée ensuite au support, par exemple au levier de l'AFM. Une des méthodes permettant d'effectuer cette liaison consiste à aimanter le levier. Ceci peut se faire par différentes techniques directes ou en collant un petit aimant à son extrémité. La méthode indirecte permet notamment un accrochage réversible. En tirant suffisamment fort, la microbille se détache du levier et celui-ci est libre pour accrocher une nouvelle microbille.

Cette méthode permet de séquencer ou de tester en série une population hétérogène de molécules d'ADN (déjà ancrées au support mobile), en les accrochant (d'une manière réversible) l'une après l'autre à la surface de mesure (le levier de l'AFM).

Les méthodes directes nécessitent, en général, de fonctionnaliser l'ADN ou l'ARN, en particulier les extrémités 3' et 5', c'est-à-dire d'y greffer des groupements chimiques adéquats. Il est, par ailleurs, préférable de relier les deux autres extrémités libres de la molécule par une boucle afin d'éviter que les brins ne se dissocient à la fin de l'opération, pour pouvoir éventuellement la réitérer. Pour cela on peut procéder de différentes manières.

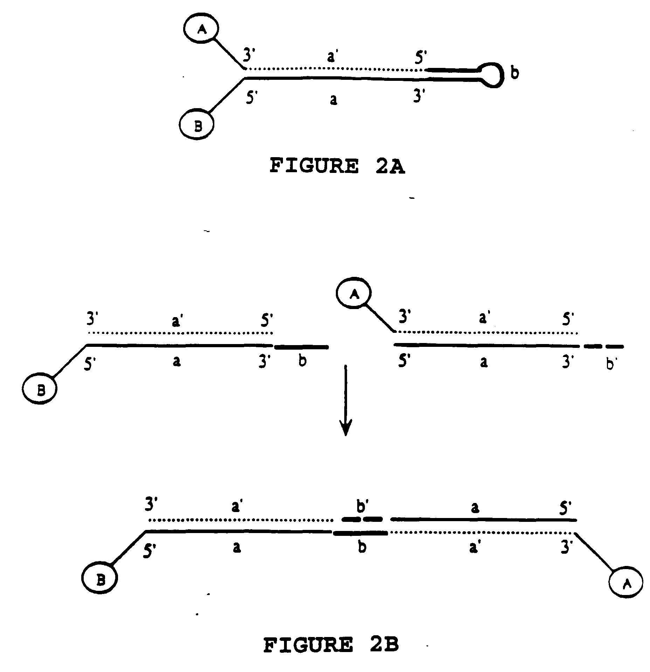

La plus simple est de fonctionnaliser à l'aide d'oligonucléotides de synthèse l'une des extrémités d'un ADN double brin avec deux fonctions différentes (biotine et amine, par exemple), qui permettent l'ancrage à deux surfaces prétraitées différentes. Les deux brins à l'autre extrémité peuvent être reliés à l'aide d'un nucléotide de synthèse partiellement apparié en boucle. On fabrique ainsi un ADN simple brin apparié à partir d'un ADN double brin (voir figure 2(a)). L'avantage de cette méthode tient à sa capacité à fonctionnaliser une population hétérogène de larges fragments d'ADN (comme on en obtient par fractionnement d'un gène ou d'un chromosome), qui peuvent ensuite être analysés simultanément. Dans ce cas, l'échantillon d'ADN est fractionné à l'aide de deux (ou plus) enzymes de restriction, ce qui permet l'obtention d'une sous-population avec deux sites de restriction différents en ses extrémités semblables sur tous les fragments. Ceci permet de traiter différemment les deux extrémités (par exemple en reliant l'une d'elle par un oligonucléotide en boucle possédant en son extrémité le site de restriction adéquat). L'inconvénient de cette méthode réside dans les contraintes stériques entre les deux groupes fonctionnels contigus qui peuvent rendre difficile l'accrochage aux surfaces.

Ce problème peut être résolu par la méthode suivante :

Un ADN double brin (symbolisé ici : a/a') est transformé en dimère aa'/a'a grâce à l'adjonction en bout de chaîne d'oligonucléotides complémentaires, comme cela est schématisé à la figure 2(b). Pour ce faire, on considère deux populations d'ADN a/a'. Dans l'une, l'extrémité 5' du brin a est fonctionnalisée par un groupement réactif A et son extrémité 3' par un oligonucléotide b. Dans l'autre, l'extrémité 3' du brin a' est fonctionnalisée par un groupement réactif B différent de A et son extrémité 5' par un oligonucléotide complémentaire de b : b'. L'hybridation et la ligation de ces deux populations produit un ADN aba'/a'b'a dont l'un des brins (aba') est fonctionnalisé en ses deux extrémités par deux groupements réactifs différents A et B permettant son ancrage à deux surfaces différentes.

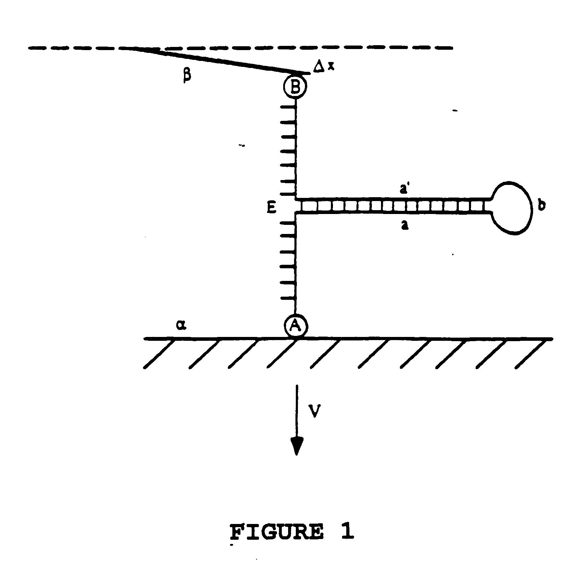

Pour obtenir la même configuration que dans la méthode précédente, il faut désolidariser les deux brins (aa' et a'a) par exemple en dénaturant l'ADN. Les deux moitiés du dimère d'ADN simple brin (aa') (accroché aux deux surfaces) étant complémentaires (par construction) elles s'apparient spontanément comme dans l'ADN initial (a/a') (voir figure 1(a)). En déplaçant l'une des surfaces, on déroule l'ADN (on "ouvre la fermeture éclair") et on mesure la force exercée sur l'autre surface gardée fixe.

Pour ce qui concerne les techniques d'ancrage proprement dites, elles sont nombreuses et dérivent des techniques d'ancrage de macromolécules (protéines, ADN, etc.) à des surfaces prétraitées commercialement disponibles. La plupart de ces techniques ont été développées pour des tests d'immunologie et lient des protéines (immunoglobulines) à des surfaces porteuses de groupements (-COOH, -NH$_2$, -OH, etc.) capables de réagir avec les extrémités carboxyle (-COOH) ou amine (-NH$_2$) des protéines.

L'ancrage covalent de l'ADN peut se faire directement, via le phosphate libre de l'extrémité 5' de la molécule qui réagit avec une amine secondaire (surface -NH Covalink™ commercialisée par Polylabo à Strasbourg) pour former une liaison covalente. On peut aussi fonctionnaliser l'ADN avec un groupement amine et procéder alors comme pour une protéine.

Il existe aussi des surfaces recouvertes de streptavidine (billes Dynal, etc.) qui permettent un ancrage quasi covalent entre la streptavidine et une molécule d'ADN biotinylée. Enfin, en greffant sur une surface (par les méthodes mentionnées précédemment) un anticorps dirigé contre la digoxigénine, on peut y ancrer un ADN fonctionnalisé par la digoxigénine. Ceci ne représente qu'un échantillon des nombreuses techniques d'ancrage possibles.

Parmi les techniques de fixation et d'ancrage, il faut citer également, par exemple, les techniques décrites dans le brevet EP 152 886 utilisant un couplage enzymatique pour la fixation de l'ADN sur un support solide tel que la cellulose.

Le brevet EP 146 815 décrit également diverses méthodes de fixation de l'ADN sur support.

De même, le brevet WO 92 16659 propose un procédé utilisant un polymère pour fixer l'ADN.

Bien entendu, l'ADN peut être fixé directement sur le support mais lorsque cela est nécessaire, en particulier en vue de limiter l'influence des surfaces, l'ADN peut être fixé au bout d'un bras inerte peptidique ou autre, comme cela est par exemple décrit dans le brevet EP 329 198.

La présente invention concerne également un intermédiaire utile pour l'étalonnage du procédé. Il s'agit d'un ADN double brin dont les extrémités 5' et 3' normalement contiguës sont, pour les unes, reliées par un oligonucléotide en boucle de manière covalente ou quasi covalente, et pour les autres, reliées à un ou plusieurs supports solides distincts.

Les exemples ci-après permettront de mettre en évidence d'autres caractéristiques et avantages de la présente invention.

Sur les figures annexées :

- La figure 1 est un schéma du procédé mettant en oeuvre un microscope à force atomique.
- La figure 2 schématise deux méthodes de fonctionnalisation de l'ADN par des groupes terminaux.
- La figure 3 schématise la construction du dimère d'ADN décrit en 2(b) appliquée à l'ADN du phage lambda.
- La figure 4 montre une image magnétique effectuée entre environ 3 et 3 μm au-dessus du substrat. Les billes du bas de l'image, blanches, sont observées sans contact direct (attraction magnétique) tandis que celles du haut, noires, le sont par contact direct (force répulsive de contact). Les billes imagées ont un diamètre de 2,8 μm.
- La figure 5 montre des approches effectuées avec le capteur de force a la verticale du centre d'une bille (a) ou du substrat vierge (b). Un champ magnétique est appliqué. Sur chaque graphe la courbe en trait continu représente l'approche depuis une position éloignée de quelques microns du substrat ; la courbe en pointillés représente le retour. En (a) l'attraction magnétique se manifeste par une montée de la courbe au-dessus de la valeur à grande distance. En (a) et (b) la répulsion (au contact) se manifeste par une baisse rapide de la valeur de la force.
- La figure 6 montre les déplacements du centre de la bille pendant un peu plus de 5 minutes ; en haut, déplacements dans le plan d'observation, en bas évolution temporelle de la position suivant X de la bille au cours du temps.
- La figure 7 montre un diagramme d'étirement d'une molécule d'ADN-λ par extension en utilisant un microscope à force atomique. Lorsque la molécule est presque complètement étendue, elle exerce une force de l'ordre de quelques nN avant de casser.

## EXEMPLE 1

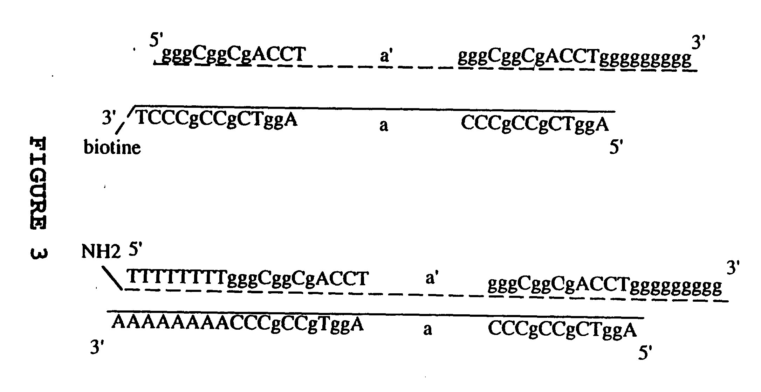

Dans cet exemple, l'ADN choisi est l'ADN du phage λ (ADN-λ) qui comprend 48502 paires de bases, dont la séquence est connue. Les deux brins a et a' de cet ADN dans sa configuration linéaire (ouverte) ont 12 bases non-appariées en leur extrémité 5'. On peut aisément fonctionnaliser les extrémités de cet ADN comme cela est schématisé sur la figure 3. Grâce à des oligonucléotides de synthèse complémentaires des extrémités non-appariées de cet ADN, les extrémités 5' du brin a et 3' du brin complémentaire a' sont l'une biotinylée, l'autre aminée. Des oligonucléotides de synthèse b et b' (en partie complémentaires de l'extrémité libre de ces ADN) sont ensuite appariés et liés. Finalement les deux ADN sont hybridés et liés. Les oligonucléotides sont indiqués en trait gras et leur séquence en italique.

Ceci fait, l'ADN fonctionnalisé est ancré (par des groupements biotine, amine ou digoxigénine) à la surface de billes de polystyrène prétraitées (commercialisées entre autres par Dynal et Rhône-Poulenc sous la marque Estapor™). Celles-ci sont recouvertes soit de streptavidine, qui a une affinité quasi covalente avec la biotine, soit d'un anticorps monoclonal permettant la liaison avec un autre anticorps dirigé contre la digoxigénine (DIG), soit encore de groupements carboxyles (COOH) qui forment une liaison covalente avec un groupement amine ($NH_2$), comme cela est décrit ci-après dans Matériels et Méthodes.

Pour tirer sur un seul brin d'un dimère d'ADN (aa'), comme cela est schématisé à la figure 1, on ancre l'une de ses extrémité à une surface plane prétraitée α via une liaison, grâce à une fonctionnalisation par un groupement amine A et on ancre l'autre extrémité à une bille magnétique Dynal B recouverte de streptavidine. Un petit aimant collé sur l'extrémité du levier d'un AFM permet de maintenir la liaison entre le levier et la bille Dynal. Les deux brins a et a' étant complémentaires, ils sont partiellement appariés. La surface α est entrainée à une vitesse v et la force de traction sur l'ADN mesurée par les déflections de la surface β qui sert de ressort (par exemple le levier d'un AFM). Les bases de l'ADN se désapparient au point E. La mesure du déplacement et de la force se fait grâce à l'AFM.

## EXEMPLE 2

## MATERIELS ET METHODES

L'ADN-λ provient de Boehringer-Mannheim. Les oligonucléotides complémentaires de l'extrémité cos de l'ADN du phage lambda ont été synthétisés (oligo1 : 5'-AGGTCGCCGCCC-3' 12-mer ; oligo2 : 5'-GGGCGGCGACCT-3' 12-mer). Tous les enzymes employés proviennent de Boehringer-Mannheim et Bio-Labs. Les nucléotides $^{32}$P proviennent de Amersham. Toutes les billes magnétiques proviennent de Dynal (Dynabeads M-280 Streptavidin: Dynabeads™ M-450 sheep anti-Mouse IgG). Les particules de latex proviennent de Rhône-Poulenc (Estapor™). L'immunoglobuline (anticorps monoclonal( anti-DIG provient de Boehringer-Mannheim. Les modules Covalink NH™ proviennent de Nunc, et des plaquettes de différents lots ont été employées. Tous les réactifs proviennent de Sigma. Le matériel marqué a été purifié sur une colonne P-6 spin™ (Bio-Rad).

## FONCTIONNALISATION DES EXTREMITES DE L'ADN

De manière à fonctionnaliser d'une façon différente les deux extrémités d'une molécule d'ADN-λ,, la méthode suivante a été employée :

### 1) Marquage de l'extrémité de la molécule avec un fragment de Klenow

0,08 nmole (2,5 µg) d'ADN-λ sont marquées à leur extrémité avec 2 unités de fragment de Klenow en présence de 2 nmoles de biotine-dUTP (ou Dig-dUTP ou amino-dUTP) et des nucléotides radioactifs, dCTP ou dGTP (3000 Ci/mmole), de manière à évaluer le rendement des différentes réactions. Les réactions sont conduites dans des conditions standards de marquage des extrémités. Les ADN marqués sont purifiés des nucléotides libres sur une colonne spin. Les ADN marqués sont fragmentés en deux fragments (45 kbp et 3 kbp) par 4 unités d'enzyme de restriction KAS1 à 37°C pendant 4 à 6 heures. Les deux fragments sont séparés sur agarose faible fusion à 1,5 %, élués et concentrés avec une colonne Elutip™. Les fragments ainsi obtenus possèdent deux extrémités différentes (une avec un 5'-phosphate et une autre avec une biotine, DIG ou un groupe amino proche de l'extrémité 3'). Ils peuvent être liés à des surfaces activées comme décrit ci-dessous ou employés pour effectuer des constructions dimères par ligation de deux fragments : l'un avec l'extrémité biotine (par exemple) et l'autre avec un groupe DIG sur l'autre extrémité.

### 2) Marquage des extrémités par des oligonucléotides modifiés

10 nmoles d'oligonucléotides homologues des deux extrémités cos de l'ADN-λ sont modifiées à leur extrémité. Tous les oligonucléotides sont phosphorylés en 5' par une réaction de Kinase. 1 nmole d'oligonucléotides est incubée à 37°C pendant 3 à 4 heures en présence de 200 nmoles d'ATP (marquées radioactivement avec 16 pmoles de $\gamma$-ATP$^{32}$ - 3000Ci/mmole) et 10 µl de Kinase-polynucléotide $T_4$. Ces oligonucléotides phosphorylés en 5' sont purifiés des nucléotides libres sur une colonne spin. Leur extrémité 3' est alors modifiée par incorporation de biotine-dUTP, DIG-dUTP ou amino-dUTP. 10 pmoles d'oligonucléotides phosphorylés en 5' sont incubés à 37°C pendant 1 à 2 heures en présence de 10 nmoles de biotine-dUTP (DIG-UTP ou amino-dUTP) et 2 unités d'enzyme terminale déoxy-transférase. Les oligonucléotides modifiés sont purifiés des nucléotides libres sur une colonne spin.

2 pmoles de ces oligonucléotides modifiés sont liées avec 0,1 pmole d'ADN-λ en présence 5 unités de ligase $T_4$ à 16°C pendant 12 heures dans un volume de réaction de 200 µl. Les oligonucléotides non liés sont séparés de l'ADN marqué par deux précipitations successives dans un mélange acétate d'ammonium/isopropanol. L'ADN est nettoyé dans de l'éthanol froid à 70 %, séché dans un "speed-vac" et mis en suspension dans de l'eau distillée.

## ANCRAGE DE LA SURFACE

Les ADN fonctionnalisés décrits précédemment peuvent avoir 4 groupes fonctionnels différents (5' phosphate, biotine, DIG et $NH_2$) lesquels sont capables de réagir avec des surfaces prétraitées disponibles dans le commerce.

### 1) Ancrage du groupe biotine aux surfaces enrobées de streptavidine

Les billes Dynal M-280 enrobées de streptavidine sont lavées trois fois dans une solution 1 x PBS (pH 7,4) + 0,1 % BSA. Les billes sont recueillies avec un concentrateur de particules magnétiques Dynal, remises en suspension dans une solution TE + 2 moles de NaCl et incubées dans un rotator avec l'ADN modifié (à différents rapports molécules d'ADN/billes) à température ambiante pendant 1 à 2 heures.

### 2) Ancrage du DIG aux surfaces enrobées d'anti-DIG

Les billes Dynal M-450 enrobées avec une IgG de mouton anti-souris sont nettoyées selon les indications données par Dynal. 100 ml d'une solution de billes (environ $4.10^7$ billes) sont incubées dans un rotator à 4°C pendant 12 heures avec 10 µg d'IgG anti-DIG (environ $10^{-10}$ mole). Les billes sont ensuite nettoyées dans une solution de PBS/BSA et remises en suspension dans le PBS. L'ADN marqué par le DIG est alors incubé avec les billes (à différents rapports) dans un rotator à température ambiante pendant 4 heures.

### 3) Ancrage des groupes amino aux surfaces acides COOH

Les particules de latex Estapor™ COOH (diamètre 1,1 et 2 µm) sont nettoyées deux fois par centrifugation dans de l'eau distillée et préactivées par du 3-diméthylaminopropyle carbodiimide (CDI). Cette étape est effectuée par incubation de O,1 mi des billes nettoyées avec O,1 mg de CDI à 50°C pendant 1 heure. Les particules préactivées sont nettoyées trois fois par centrifugation d'une solution 0,05 mole de morpholino éthane sulfonique (MES) à pH 5,5. Les billes précipitées sont remises en suspension dans 100 µl de TPG (0,05 mole $NaH_2PO_4$, $2H_2O$; O,1 % NaCl ; 2 % de gélatine; pH 6,6).

Les billes peuvent alors former des liaisons covalentes avec les groupes amino présents dans la protéine et les groupes amino des ADN modifiés. 100 µl d'une solution de latex (environ $10^9$ billes) sont incubés en présence de 0,5 mg d'avidine (ou 0,5 mg de protéine A ou 0,25 pmole d'oligo2 amino-modifié) à 50°C pendant 1 heure et ensuite maintenus en rotation à température ambiante pendant 12 heures. Les billes liées sont ensuite nettoyées 3 fois avec un tampon TPG, resuspendues dans 100 µl de TPG et maintenues à 4°C. Les billes enrobées d'avidine sont liées aux ADN modifiés par de la biotine, comme décrit précédemment. Les billes couvertes de protéine A sont enrobées avec une IgG anti-DIG, par incubation avec 0,1 mg/ml d'anticorps à température ambiante pendant 12 heures. Enfin, la liaison avec l'ADN DIG-modifié est effectuée comme décrit précédemment.

4) Ancrage des groupes 5' phosphate aux surfaces activées par une amine secondaire (NH) (Nunc Covalink™ modules)

Différentes quantités d'ADN-λ (de 50 ng à 0,005 ng) sont dissoutes dans 75 µl d'une solution 10 mmoles de 1-méthylimidazole (1-MeIM) et 25 µl de 0,2 M de 1-éthyl-3-diméthylaminopropyle-carbodiimide (EDC). Cette solution est chargée dans un puits Nunc Covalink™. Les modules sont incubés à 50°C pendant 5 à 6 heures, suivi par 3 nettoyages avec une solution 5 x SSC, 0,25 % SDS (préchauffée à 50°C) et 3 nettoyages avec 0,1 x TE.
De manière à éviter les liaisons non spécifiques des billes préactivées à la surface du puits, une solution BSA à 0,1 % est ajoutée et incubée à température ambiante pendant 12 heures. Les puits sont nettoyés 3 à 5 fois avec 0,1 x TE. Les ADN marqués par la biotine sont dénaturés dans une solution de NaOH 0,25 mole de manière à libérer les extrémités 3' de la biotine. Les billes M-280 enrobées de streptavidine sont ensuite ajoutées pour se lier avec l'extrémité biotine et sont incubées à température ambiante pendant 0,5 heure. Les billes non liées sont récoltées par un moyen magnétique. L'ADN lié par son extrémité 5' phosphate à la surface du puits et par son extrémité 3' marqué par la biotine à la surface des billes est maintenant près pour manipulation.

## AIMANTATION DU LEVIER

De manière à prélever sélectivement les billes paramagnétiques M-280 attachées, le levier AFM est aimanté comme suit : à l'aide d'une micropipette à l'extrémité d'un micromanipulateur (Leitz) et sous microscope (Reichert-Jung Polyvar), une petite goutte d'un adhésif durcissable aux ultraviolets (Norland Optical Adhesive) est déposée à la pointe du cantilever AFM (Park). Une petite particule magnétique (rayon environ 7 µm) (une bille de cobalt ou un grain d'aimant SmCo pulvérisé) est prise avec la micropipette, placée sur la goutte de colle et polymérisée par éclairage UV pendant environ 20 minutes. Le levier aimanté est employé pour prélever les particules liées à la surface covalink, placé dans un champ magnétique sous un microscope optique inversé (Nikon) ou un AFM (Park Scientific Instruments, USA).

## EXEMPLE 3

## RESULTATS

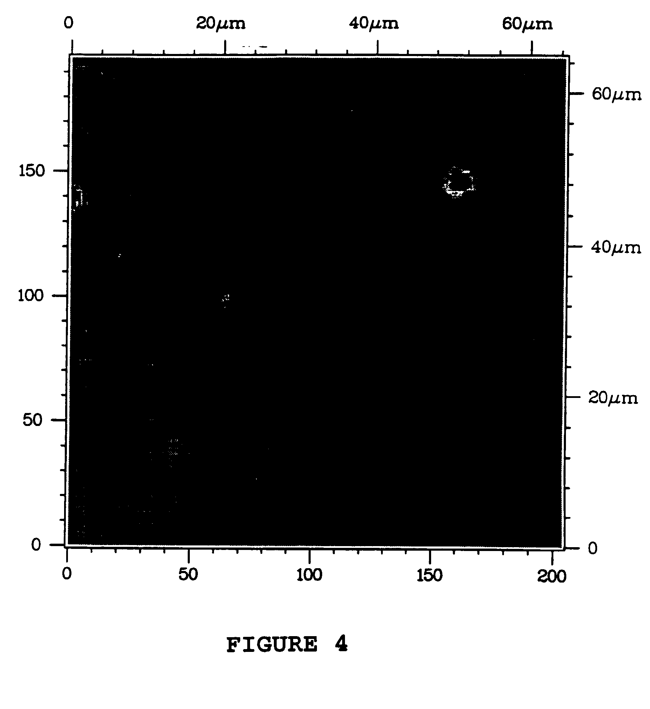

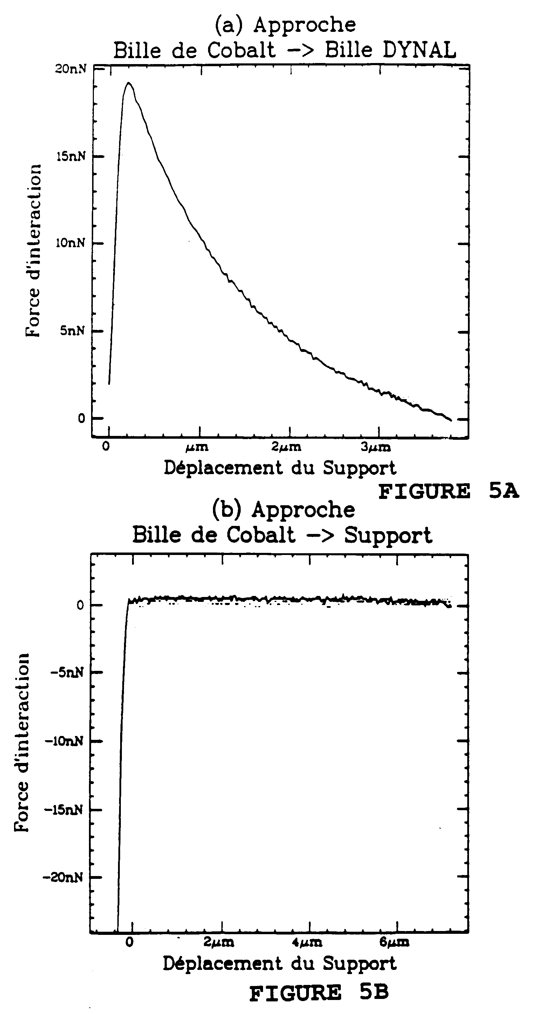

On utilise un microscope à force atomique (AFM) essentiellement constitué d'un petit levier élastique (cantilever) de 100 à 200 µm de long et de 0,6 µm d'épaisseur. La position de l'extrémité fixe du levier est contrôlée à quelques $10^{-11}$ m par rapport à l'échantillon au moyen d'un tube piézoélectrique. La déflection de l'extrémité mobile, mesurée optiquement permet de détecter la force appliquée avec une sensibilité avoisinant $10^{-12}$ N. A cette dernière extrémité, on a remplacé la sonde classique de l'AFM (une pointe de nitrure de silicium de quelques dizaines de nanomètres de rayon de courbure) par une bille de cobalt, ferromagnétique de 7 µm de diamètre. Cet ensemble constitue un capteur de forces magnétiques. Ce capteur magnétique peut être attiré par une bille Dynal de 2,8 µm fixée sur un substrat solide, ce qui donne lieu à une déflection du ressort. Inversement, le capteur peut attirer une bille Dynal en solution ou partiellement libre à l'extrémité d'un brin d'ADN. Il n'y a alors pas ou peu de déflection et la bille vient se coller spontanément sur le capteur. Le premier type d'interactions est mesurable directement par l'AFM, le second est observable visuellement en microscopie optique.
Les billes Dynal peuvent être rendues solidaires du substrat activé (modules Nunc-Covalink™) par liaison biochimique directe ou par adhésion non-spécifique. Cette adhésion est renforcée par les gradients de champ d'un petit aimant situé à quelques millimètres sous le substrat. La force d'attraction entre le capteur et une bille, maximale à la limite du contact, atteint $10^{-8}$ à $10^{-9}$ N. En l'absence de champ magnétique la portée de l'interaction (dipôle permanent - dipôle induit) est très faible par rapport aux diamètres des billes et est donc difficilement mesurable. Par contre, un petit champ sature l'aimantation des dipôles induits, augmentant ainsi la portée qui atteint 4 µ environ. On peut alors réaliser des images en balayant avec le capteur un plan situé à 1 ou 2 µ au-dessus de la bille (voir figure 4). Ceci permet une localisation très fine des billes dont on peut alors s'approcher. La figure 5 montre des approches effectuées à la verticale du centre d'une bille et/ou du support vierge. On voit en (a), de droite à gauche, l'attraction magnétique à distance puis le contact du capteur avec la bille. En (b), seul le contact du capteur avec le support est détecté. En l'absence de champ, on n'observe aucune attraction significative à cette échelle mais seulement les interactions de contact.

Des billes libres peuvent être micromanipulées avec la bille de cobalt du capteur de l'AFM. Ceci n'est pas actuellement réalisable sous l'AFM même, faute d'accès optique pour le contrôle de l'opération, mais peut-être simulé au moyen d'un capteur attaché à un micromanipulateur dans le champ d'un microscope optique inversé. Un AFM intégré à un microscope inversé est à l'étude. On observe essentiellement que les billes libres sont attirées par le capteur dans un rayon de 1 ou 2 µ et viennent s'y coller. Ceci est le moyen de capture le plus élémentaire que nous comptons appliquer au système bille-ADN. La force de liaison entre les deux billes est la somme de la force d'attraction magnétique décrite ci-dessus et d'une force de contact fonction essentiellement des états des surfaces : la liaison est assez forte sur un capteur non traité et ne peut être rompue aisément. Toutefois, si le capteur est trempé dans une solution de serum d'albumine bovine (BSA) (environ 2 %) la force de contact est fortement diminée et la bille peut être relachée par une accélération brusque du capteur ou un bref frottement contre le substrat.

Le dispositif opérationnel d'analyse de l'ADN en vue de son séquençage est typiquement constitué autour du substrat transparent (Nunc-Covalink) où sont greffées les molécules d'ADN. Ce substrat est solidaire d'un système piezoélectrique ou équivalent qui le déplace dans les trois directions d'espace. Au-dessus, un capteur de force magnétique (AFM) permet les mesures quantitatives. Au-dessous un microscope optique permet d'observer la surface et le capteur de force dans leur mouvement relatif. Enfin, l'ensemble est dans le champ d'un électro-aimant qui, selon le champ appliqué, peut plaquer les billes à la surface, permettant ainsi l'imagerie magnétique, les éloigner de la surface (élimination des billes non greffées pour faciliter le choix de l'opérateur) ou, avec un champ nul, laisser librement évoluer l'ADN afin qu'il vienne se coller au capteur. Dans cette configuration, on peut alors réaliser la mesure quantitative des forces et le séquençage de l'ADN.

## EXEMPLE 4

Cet exemple a pour objet des mesures quantitatives de la force sur la molécule lors d'une traction.

Pour se faire, il faut réaliser trois conditions : attacher des billes magnétiques à une plaque de verre par des molécules d'ADN, pouvoir déterminer le nombre de molécules qui relient une bille à la plaque de verre afin de choisir celles qui le sont par une bille, finalement, mesurer la force de traction sur la molécule en utilisant un microscope à force atomique (AFM).

L'accrochage de la molécule d'ADN suit un protocole voisin de celui décrit ci-dessus. L'attachement de chacune des extrémités de la molécule d'ADN se fait au cours d'étapes distinctes ; ceci afin d'éviter que les deux extrémités d'une seule molécule ne se lient, soit à la bille, soir à la surface de verré en formant une boucle. L'extrémité où l'on souhaite attacher la bille magnétique est d'abord greffée sélectivement de la biotine, qui se liera à la streptavidine dont est recouverte la bille magnétique. La seconde extrémité est attachée à une molécule Digoxygénine (DIG-dUTP) qui forme un lien avec son anticorps anti-DIG dont on a recouvert une plaque de verre préalablement traitée à la protéine A.

### Etirement d'une molécule d'ADN

En approchant une pointe aimantée constituée d'une bille de cobalt, à l'aide d'un micromanipulateur, on a pu vérifier qu'un certain nombre de billes sont captives et ne peuvent s'écarter de leur point d'ancrage au-delà d'une distance de 15 µm, ce qui correspond précisément à la longueur de la molécule d'ADN utilisée.

### Caractérisation de l'échantillon

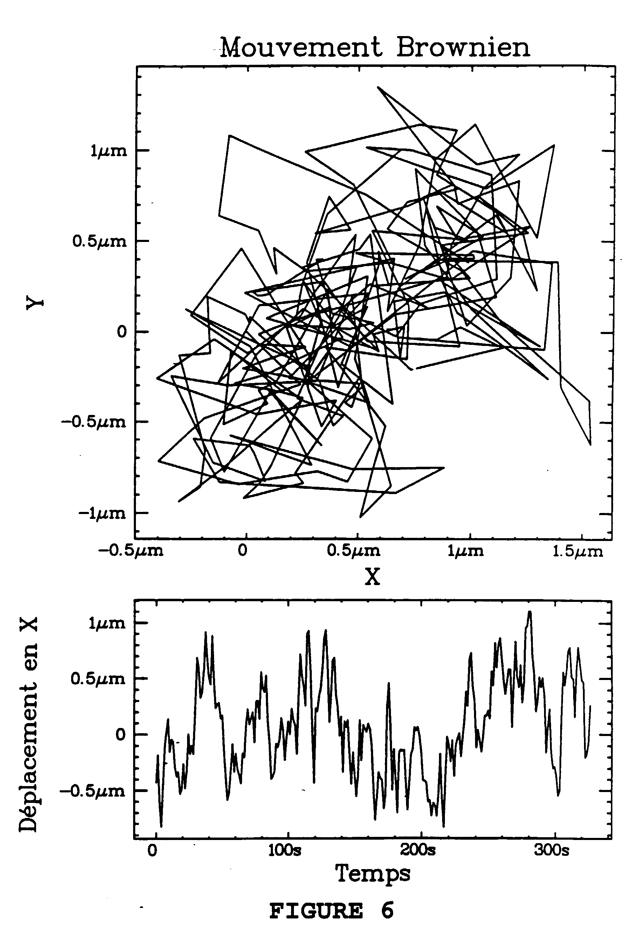

Lors de la préparation de l'échantillon, le nombre de molécule d'ADN qui relient une bille à la plaque de verre peut varier, il faut donc déterminer a posteriori le nombre de molécules impliquées dans la liaison. On a mis au point un dispositif permettant cette détermination de façon non perturbative : on mesure l'amplitude des mouvements brownien de la bille et on en déduit le nombre de molécules qui la relient à la plaque de verre. En effet, plus ce nombre de molécules est grand plus la bille est attachée solidement à la surface et moins grande est l'amplitude de ces fluctuations. On a reproduit sur la figure 6 l'allure des fluctuations observées correspondant au cas où la bille est reliée par une seule molécule d'ADN.

Pour déterminer le nombre de molécule, on mesure $\langle x^2 \rangle$ et on utilise l'expression :

$$nk \langle x^2 \rangle = k_B T$$

où k est la raideur d'une molécule d'ADN, $k_B$ est la constante de Boltzmann, T est la température et n est le nombre de molécule.

Brisure d'une molécule d'ADN

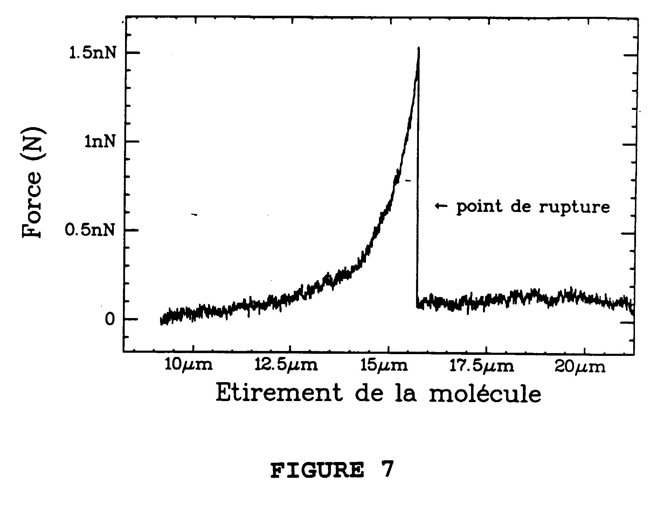

En augmentant l'aimantation de la bille de colbalt collée sur la pointe du micromanipulateur, on peut alors casser la molécule d'ADN en écartant la bille au-delà de 15 µm. En répétant cette expérience avec le microscope à force atomique on a mesuré la force de tension sur la molécule au moment de la rupture de celle-ci (figure 7).

## Revendications

**1.** Procédé de détermination d'une séquence d'ADN ou d'ARN, caractérisé en ce qu'on détermine l'énergie d'appariement ou de désappariement entre chaque paire de bases de l'ADN ou d'un hybride ARN/ADN double brin correspondant à la séquence d'ADN ou d'ARN à déterminer et en ce que l'on compare cette énergie à une valeur prédéterminée.

**2.** Procédé selon la revendication 1, caractérisé en ce que ladite valeur prédéterminée correspond à l'énergie d'appariement ou de désappariement d'une paire de bases connue en présence de bases avoisinantes données.

**3.** Procédé pour la mise en évidence de la présence ou de l'absence d'une séquence d'ADN ou d'ARN déterminée dans un échantillon à tester, caractérisé en ce qu'on détermine ses éléments essentiels en déterminant l'énergie d'appariement ou de désappariement de chaque paire de bases de ladite séquence connue pour en faire une empreinte et on contrôle si ladite séquence d'ADN ou d'ARN, présente dans ledit échantillon, possède la même empreinte ou non.

**4.** Procédé pour la mise en évidence de la présence ou de l'absence d'une séquence d'ADN ou d'ARN déterminée dans un échantillon à tester, caractérisé en ce qu'on détermine l'énergie globale nécessaire pour désapparier un ADN comportant une partie double brin ou un hybride ADN/ARN correspondant à la séquence d'ADN ou d'ARN dont on souhaite mettre en évidence la présence ou l'absence et on compare l'énergie globale à une valeur de seuil de cette énergie correspondant à ladite séquence totalement appariée.

**5.** Procédé selon l'une des revendications 1 à 4, caractérisé en ce qu'on détermine l'énergie de désappariement entre chaque paire de bases de l'ADN ou d'hybride ADN/ARN par fixation d'au moins une base de chaque brin de l'ADN ou de l'hybride ADN/ARN sur un support et éloignement des supports afin de désapparier, l'une après l'autre, chaque paire de bases en mesurant à chaque désappariement l'énergie nécessaire au désappariement et en la comparant avec des valeurs prédéterminées.

**6.** Procédé selon l'une des revendications 1 à 4, caractérisé en ce qu'on détermine l'énergie d'appariement entre chaque paire de bases de l'ADN ou d'hybride ADN/ARN par fixation d'au moins une base de chaque brin de l'ADN ou de l'hybride ADN/ARN sur un support et rapprochement des supports afin d'apparier chaque paire de bases en mesurant à chaque appariement l'énergie nécessaire à l'appariement et en la comparant avec des valeurs prédéterminées.

**7.** Procédé selon l'une des revendications 1 à 6, caractérisé en ce qu'il est mis en oeuvre pour le séquençage d'un ADN ou d'un hybride ADN/ARN de constitution non déterminée, les valeurs prédéterminées étant mesurées au préalable pour chaque paire de bases en fonction de son environnement.

**8.** Procédé selon l'une des revendications 1 à 7, caractérisé en ce que les extrémités non fixées sur supports de l'ADN double brin ou d'un hybride, avant appariement ou désappariement, sont reliées entre elles de manière covalente ou quasi covalente.

**9.** Procédé selon l'une des revendications 1 à 8, caractérisé en ce que la séquence d'ADN double brin ou d'hybride ADN/ARN, après désappariement des deux brins, est réappariée puis de nouveau arrachée à plusieurs reprises afin d'accumuler les mesures et augmenter le rapport signal/bruit.

**10.** Procédé selon l'une des revendications 1 à 9, caractérisé en ce que chaque brin d'ADN ou de l'hybride est fixé par plusieurs bases contiguës.

**11.** Procédé selon l'une des revendications 1 à 10, caractérisé en ce que l'une des bases de l'un des brins de l'ADN ou d'un hybride est fixée directement ou indirectement sur une surface mobile et en ce qu'une base de l'autre brin d'ADN ou d'hybride est fixée sur un capteur de force et en ce que l'on éloigne la surface mobile en déterminant la valeur des forces mesurées par le capteur de force lors du désappariement des bases.

12. Procédé selon la revendication 11, caractérisé en ce que pour fixer indirectement l'une des bases sur une surface mobile, on utilise un aimant fixé sur la surface et une particule magnétisable fixée sur une base.

13. Procédé selon la revendication 12, caractérisé en ce que le capteur de force est le levier d'un microscope à force atomique et la surface mobile est un dispositif de déplacement nanométrique.

14. Procédé selon l'une des revendications 11 à 13, caractérisé en ce que le capteur de force est un aimant dont on asservit la position par un champ magnétique.

15. Procédé selon l'une des revendications 11 à 14, caractérisé en ce que le capteur de force est un levier ou une membrane associé à un capteur de déplacement.

16. Procédé selon l'une des revendications 1 à 15, caractérisé en ce qu'on place un échantillon d'ADN ou d'ARN à tester, préalablement fixé sur un support, pour la présence d'une séquence spécifique, dans un récipient contenant la séquence complémentaire de ladite séquence spécifique, fixée elle-aussi sur un support, et en ce qu'après incubation on détermine l'énergie de liaison entre l'ADN double brin ou l'hybride.

17. Application du procédé selon l'une des revendications 1,2 et 5 à 16 au séquençage d'un ADN inconnu.

18. Application du procédé selon l'une des revendications 1 à 16 à la mise en évidence de la présence ou de l'absence d'une séquence d'ADN déterminée dans un échantillon à tester.

19. ADN double brin ou hybride ADN/ARN double brin destiné à la mise en oeuvre du procédé selon l'une des revendications 1 à 16, caractérisé en ce que deux des extrémités libres 5' et 3' sont reliées par un oligonucléotide en boucle de manière covalente ou quasi-covalente, les deux autres extrémités libres étant fonctionnalisées par des groupements chimiques distincts.

**Claims**

1. Method for the determination of a DNA or RNA sequence, characterized in that the energy of pairing or of unpairing between each base pair of the double-stranded DNA or of a double-stranded RNA/DNA hybrid corresponding to the DNA or RNA sequence to be determined is determined, and in that this energy is compared with a predetermined value.

2. Method according to Claim 1, characterized in that the said predetermined value corresponds to the pairing or unpairing energy of a known base pair in the presence of given adjacent bases.

3. Method for demonstrating the presence or the absence of a determined DNA or RNA sequence in a test sample, characterized in that its essential features are determined by determining the pairing or unpairing energy of each base pair of the said known sequence to make a fingerprint thereof and it is checked whether the said DNA or RNA sequence, present in the said sample, has the same fingerprint or not.

4. Method for demonstrating the presence or the absence of a determined DNA or RNA sequence in a test sample, characterized in that the global energy necessary for unpairing a DNA containing a double-stranded part or a DNA/RNA hybrid corresponding to the DNA or RNA sequence whose presence or absence it is wished to demonstrate is determined and the global energy is compared with a threshold value of this energy corresponding to the said totally paired sequence.

5. Method according to one of Claims 1 to 4, characterized in that the energy of unpairing between each base pair of the DNA or DNA/RNA hybrid is determined by attaching at least one base of each strand of the DNA or of the DNA/RNA hybrid to a support and moving away the supports so as to unpair, one after another, each base pair while measuring at each unpairing the energy needed for unpairing and comparing it with predetermined values.

6. Method according to one of Claims 1 to 4, characterized in that the energy of pairing between each base pair of the DNA or DNA/RNA hybrid is determined by attaching at least one base of each strand of the DNA or of the DNA/RNA hybrid to a support and bringing the supports together so as to pair each base pair while measuring at each pairing the energy needed for pairing and comparing it with predetermined values.

7. Method according to one of Claims 1 to 6, characterized in that it is employed for the sequencing of a DNA or of a DNA/RNA hybrid of undetermined constitution, the predetermined values being measured beforehand for each base pair according to its environment.

8. Method according to one of Claims 1 to 7, characterized in that the ends of the double-stranded DNA or of a hybrid which are not attached to supports, before pairing or unpairing, are joined to one another covalently or quasi-covalently.

9. Method according to one of Claims 1 to 8, characterized in that the double-stranded DNA or DNA/RNA hybrid sequence, after unpairing of the two strands, is re-paired and then pulled apart again several times so as to accumulate the measurements and increase the signal/noise ratio.

10. Method according to one of Claims 1 to 9, characterized in that each strand of DNA or of the hybrid is attached by several adjacent bases.

11. Method according to one of Claims 1 to 10, characterized in that one of the bases of one of the strands of the DNA or of a hybrid is attached directly or indirectly to a movable surface, and in that a base of the other strand of DNA or of hybrid is attached to a force sensor, and in that the movable surface is moved away while determining the value of the forces measured by the force sensor on unpairing of the bases.

12. Method according to Claim 11, characterized in that, to attach one of the bases indirectly to a movable surface, a magnet attached to the surface and a magnetizable particle attached to a base are used.

13. Method according to Claim 12, characterized in that the force sensor is the lever of an atomic force microscope and the movable surface is a nanometric movement device.

14. Method according to one of Claims 11 to 13, characterized in that the force sensor is a magnet whose position is controlled by a magnetic field.

15. Method according to one of Claims 11 to 14, characterized in that the force sensor is a lever or a membrane in combination with a movement sensor.

16. Method according to one of Claims 1 to 15, characterized in that a DNA or RNA sample to be tested, previously attached to a support, for the presence of a specific sequence is placed in a vessel containing the sequence complementary to the said specific sequence, which is itself also attached to a support, and in that, after incubation, the energy of bonding between the double-stranded DNA or the hybrid is determined.

17. Application of the method according to one of Claims 1, 2 and 5 to 16 to the sequencing of an unknown DNA.

18. Application of the method according to one of Claims 1 to 16 to the demonstration of the presence or abscene of a particular DNA sequence in a test sample.

19. Double-stranded DNA or double-stranded hybrid DNA/RNA intended for the implementation of the method according to one of Claims 1 to 16, characterized in that two of the free 5' and 3' ends are joined covalently or quasi-covalently by a cyclic oligonucleotide, the two other free ends being functionalized by different chemical groups.

**Patentansprüche**

1. Verfahren zur Bestimmung einer DNA- oder RNA-Sequenz, dadurch charakterisiert, daß man die Paarungs- oder Trennungsenergie zwischen jedem Basenpaar der DNA oder eines Doppelstrang-RNA/DNA-Hybrids bestimmt, das der zu bestimmenden DNA- oder RNA-Sequenz entspricht, und daß man diese Energie mit einem vorherbestimmten Wert vergleicht.

2. Verfahren nach Anspruch 1, dadurch charakterisiert, daß der vorherbestimmte Wert der Paarungs- oder Trennungsenergie eines bekannten Basenpaares in Gegenwart von gegebenen Nachbarbasen entspricht.

3. Verfahren zum Nachweis der Anwesenheit oder Abwesenheit einer bestimmten DNA- oder RNA-Sequenz in einer zu testenden Probe, dadurch charakterisiert, daß man ihre wesentlichen Elemente durch Bestimmung der Paarungs- oder Trennungsenergie jedes Basenpaares der bekannten Sequenz bestimmt, um daraus einen Fingerabdruck zu erhalten, und kontrolliert, ob die DNA- oder RNA-Sequenz, die in der Probe vorhanden ist, denselben Fingerabdruck besitzt oder nicht.

4. Verfahren zum Nachweis der Anwesenheit oder Abwesenheit einer bestimmten DNA- oder RNA-Sequenz in einer zu testenden Probe, dadurch charakterisiert, daß man die Gesamtenergie bestimmt, die zur Trennung einer DNA notwendig ist, welche einen Doppelstrangteil oder ein DNA/RNA-Hybrid umfaßt, entsprechend der DNA- oder RNA-Sequenz, deren Vorhandensein oder Abwesenheit man nachweisen möchte, und man die Gesamtenergie mit einem Schwellenwert dieser Energie vergleicht, der der vollständig gepaarten Sequenz entspricht.

5. Verfahren nach einem der Ansprüche 1 bis 4, dadurch charakterisiert, daß man die Trennungsenergie zwischenjedem Basenpaar der DNA oder des DNA/RNA-Hybrids durch Fixierung mindestens einer Base jedes DNA-Strangs oder des DNA/RNA-Hybrids auf einem Träger und Entfernen der Träger zur schrittweisen Trennung jedes Basenpaars bestimmt, indem bei jeder Trennung die zur Trennung notwendige Energie gemessen und mit vorherbestimmten Werten verglichen wird.

6. Verfahren nach einem der Ansprüche 1 bis 4, dadurch charakterisiert, daß man die Paarungsenergie zwischen jedem Basenpaar von DNA oder einem DNA/RNA-Hybrid durch Fixierung von mindestens einer Base jedes DNA-Strangs oder des DNA/RNA-Hybrids auf einem Träger und Annäherung der Träger bestimmt, um jedes Basenpaar zu paaren, indem bei jeder Paarung die notwendige Energie zur Paarung gemessen und mit vorherbestimmten Werten verglichen wird.

7. Verfahren nach einem der Ansprüche 1 bis 6, dadurch charakterisiert, daß es für die Sequenzierung einer DNA oder eines DNA/RNA-Hybrids mit unbestimmter Konstitution durchgeführt wird, wobei die vorherbestimmten Werte zuvor für jedes Basenpaar als Funktion seiner Umgebung gemessen werden.

8. Verfahren nach einem der Ansprüche 1 bis 7, dadurch charakterisiert, daß die nicht auf Trägern fixierten Enden der Doppelstrang-DNA oder eines Hybrids vor der Paarung oder Trennung miteinander auf kovalente oder quasi-kovalente Weise gebunden werden.

9. Verfahren nach einem der Ansprüche 1 bis 8, dadurch charakterisiert, daß die Sequenz der Doppelstrang-DNA oder des DNA/RNA-Hybrids nach Trennung der beiden Stränge in mehreren Wiederholungen erneut gepaart und anschließend erneut getrennt werden, um Meßwerte zu akkumulieren und das Verhältnis Signal/Rauschen zu verbessern.

10. Verfahren nach einem der Ansprüche 1 bis 9, dadurch charakterisiert, daß jeder DNA- oder Hybrid-Strang durch mehrere aufeinanderfolgende Basen fixiert ist.

11. Verfahren nach einem der Ansprüche 1 bis 10, dadurch charakterisiert, daß eine der Basen des einen Strangs der DNA oder eines Hybrids direkt oder indirekt auf einer mobilen Oberfläche fixiert ist, und daß eine Base des anderen DNA-Strangs oder -Hybrids an einem Kraftmeßfühler fixiert ist und daß man die mobile Oberfläche entfernt, während der Wert der durch den Kraftmeßfühler gemessenen Kräfte während der Trennung der Basen bestimmt wird.

12. Verfahren nach Anspruch 11, dadurch charakterisiert, daß man zur indirekten Fixierung einer Base auf einer mobilen Oberfläche einen auf der Oberfläche fixierten Magneten und eine auf einer Base fixierten magnetisierbaren Partikel verwendet.

13. Verfahren nach Anspruch 12, dadurch charakterisiert, daß der Kraftmeßfühler die Meßspitze eines Atomkraftmikroskops (atomique force microscope, AFM) ist und die mobile Oberfläche eine Vorrichtung zur Nanometer-Verschiebung ist.

14. Verfahren nach einem der Ansprüche 11 bis 13, dadurch charakterisiert, daß der Kraftmeßfühler ein Magnet ist, dessen Position durch ein Magnetfeld festgelegt wird.

15. Verfahren nach einem der Ansprüche 11 bis 14, dadurch charakterisiert, daß der Kraftmeßfühler ein Meßfühler oder eine Membran ist, die mit einem Verschiebungsmeßfühler verbunden sind.

16. Verfahren nach einem der Ansprüche 1 bis 15, dadurch charakterisiert, daß man die auf das Vorhandensein einer spezifischen Sequenz zu testende zuvor auf einem Träger fixierte DNA- oder RNA-Probe in einen Behälter gibt, der die komplimentäre Sequenz der spezifischen Sequenz enthält, ebenfalls auf einem Träger fixiert, und daß man nach Inkubation die Bindungsenergie zwischen der Doppelstrang-DNA oder der Hybrid-DNA bestimmt.

17. Anwendung des Verfahren nach einem der Ansprüche 1, 2 und 5 bis 16 bei der Sequenzierung einer unbekannten DNA.

18. Anwendung des Verfahrens nach einem der Ansprüche 1 bis 16 zum Nachweis der Anwesenheit oder Abwesenheit einer bestimmten DNA-Sequenz in einer zu testenden Probe.

19. Doppelstrang-DNA oder Doppelstrang-DNA/RNA-Hybrid, bestimmt zur Durchführung des Verfahrens nach einem der Ansprüche 1 bis 16, dadurch charakterisiert, daß die beiden freien 5'- und 3'-Enden auf kovalente oder quasi-kovalente Weise durch ein Ring-Oligonukleotid verbunden sind, wobei die beiden anderen freien Enden durch bestimmte chemische Gruppen funktionalisiert sind.

**FIGURE 1**

**FIGURE 2A**

**FIGURE 2B**

**FIGURE 3**

5' gggCggCgACCT _ _ _ _ a' _ _ _ _ gggCggCgACCTgggggggg 3'

3' /TCCCgCCgCTggA a CCCgCCgCTggA
biotine 5'

NH2 5'
\ TTTTTTTgggCggCgACCT _ _ _ a' _ _ _ gggCggCgACCTgggggggg 3'

AAAAAAAACCCgCCgTggA a CCCgCCgCTggA
3' 5'

FIGURE 4

(a) Approche
Bille de Cobalt -> Bille DYNAL

Force d'interaction

20nN

15nN

10nN

5nN

0

0  μm  2μm  3μm

Déplacement du Support

**FIGURE 5A**

(b) Approche
Bille de Cobalt -> Support

Force d'interaction

0

-5nN

-10nN

-15nN

-20nN

0  2μm  4μm  6μm

Déplacement du Support

**FIGURE 5B**

Mouvement Brownien

FIGURE 6

FIGURE 7